# EUROPEAN PATENT APPLICATION

(11) **EP 1 932 521 A1**
(43) Date of publication of application: **18.06.2008**
(21) Application number: 06126261.4
(22) Date of filing: 15.12.2006
(51) Int. Cl.: A61K 31/07, A61K 31/202, A61K 31/30, A61K 31/315, A61K 31/355, A61K 31/375, A61P 27/02

(54) **Nutritional supplement composition for treatment of ocular diseases**

(71) Applicant: Novartis AG, 4056 Basel (CH)
(72) Inventor: The designation of the inventor has not yet been filed
(74) Representative: von Sprecher, Georg

(57) **Abstract**

The present invention relates to an antioxidant and lutein supplement composition which inter alia decreases visual acuity loss by reducing the risk of developing late stage or advanced age- related macular degeneration in people with early age-related macular degeneration.

## Description

The present invention relates to a nutritional or dietary supplement composition that strengthens and promotes retinal health through the prevention, stabilization, reversal and/or treatment of visual acuity loss in people with certain ocular diseases. More specifically, the present invention relates to an antioxidant and lutein supplement composition that decreases visual acuity loss by reducing the risk of developing late stage or advanced age- related macular degeneration in people with early age-related macular degeneration.

Accordingly, it is an object of the present invention to provide a nutritional or dietary supplement composition effective in the prevention, stabilization, reversal and/or treatment of macular degeneration and/or visual acuity loss.

Another object of the present invention is to provide a safe nutritional or dietary supplement composition for the prevention, stabilization, reversal and/or treatment of macular degeneration and/or visual acuity loss, in particular in accordance to all specified examples and claims of the present disclosure.

Another object of the present invention is to provide an effective and safe method of preventing, stabilizing, reversing and/or treating macular degeneration and/or visual acuity loss.

Another object of the present invention is to provide a method of manufacturing a safe nutritional or dietary supplement composition for the prevention, stabilization, reversal and/or treatment of macular degeneration and/or visual acuity loss.
The following detailed description is provided to enable any person skilled in the art to which the present invention pertains to make and use the same, and sets forth the best mode contemplated by the inventors of carrying out the subject invention.

The preferred nutritional or dietary supplement composition of the present invention is a formulation of essential ingredients preferably in quantities set forth below, e.g. in Table 1, to be ingested daily.

**Table 1**

| | |
|---|---|
| Each composition, e.g. in the form of a capsule, contains in a preferred embodiment the following ingredients: | |
| 240 mg Fish oil with 70% DHA | label 160 mg Omega 3 fatty acids |
| Docosahexaeonic acid (DHA) | label 130 mg DHA |
| Eicosapentaenoic acid (EPA) as part of fish oil | label 11 mg |
| 91.5mg Calcium ascorbate | label 60 mg Vitamin C |
| 31.343mg d,l Alpha-tocopheryl acetate | label 20 mg Vitamin E |
| 55mg Lutein 20% in safflower oil | label 10 mg Lutein |
| 12.447mg Zinc oxide | label 10 mg Zinc |
| 0.45mg Cupric carbonate basic | label 0.25 mg Copper |
| Zeaxanthin as part of Lutein | label 0.8 mg |

The above composition may contain other excipients for the production of a targeted galenic formulation, e.g. for making a shell of a capsule or the like.

The preferred daily dosage of the subject composition as specified above may be administered in the form of one or more dosage units, e.g. capsules. Most preferably the daily dosage of the subject composition is provided in the form of one capsules taken three times daily, for a total of 3 capsules a day, or in the form of 1 capsule taken twice daily, for a total of 2 capsules a day. Compared to taking the total daily dose once a day, twice or three times daily dosing in one or more capsules per dose provides improved absorption and better maintenance of blood levels of the essential ingredients.

Compositions, e.g. capsules of the preferred formulation of the subject composition may contain larger or smaller quantities of essential ingredients per composition, e.g. capsule than the minimum quantities per composition, e.g.capsule, specified above.
Larger quantities of essential ingredients, to compensate for some degradation which may occur over time, in particular if the essential ingredient is an antioxidant, e.g. vitamin C, E, or lutein. Smaller quantities of essential ingredients, for example, to take into account the food intake situation of a patient / animal.

Typically, the listed quantities of ingredients as contained in a composition of the present invention may typically range from 5 % below the indicated quantities of each ingredient up to 5% above the indicated quantities of each ingredient. Therefore, an addressed concentration may encompass +/- 5% by weight of an addressed and listed ingredient, unless designated differently. For illustration purposes, 10 mg vitamin E may for example encompass a range from 9.5 - 10.5 mg vitamin E.
By providing larger quantities of essential ingredients in each composition unit, e.g. capsule, one is ensured that even with ingredient degradation, the full amount of the ingredient amount specified on the composition unit sale label is provided upon oral administration of the composition unit through to the specified expiration date of the composition unit . Another consideration in formulating the subject composition is that depending on the source and/or manufacturing process of the individual ingredients, individual ingredient degradation rates may vary. Accordingly, the specific formulation of the subject composition will vary depending on the sources of the individual ingredients and the specified length of product shelf life before expiration. Typically, the product shelf life for nutritional or dietary supplements is approximately two to three years. Tablet formulations may also vary somewhat depending on slight deviations from manufacturing specifications within controlled tolerance ranges as customary within the field of art.
Variations contemplated in administering the subject composition to humans or animals include, but are not limited to, providing time-release compositions or compositions manufactured to be administered as a single dose or as other multiple part dosages. Additionally, alternative avenues of administration besides oral administration are contemplated herein such as for example, but not limited to, intraperitoneal, intravenous, subcutaneous, sublingual, transcutaneous, intramuscular or like forms of administration.

The preferred route of administration is the oral route.

As used herein the terms tablet, capsule, drageé, pellet, suppository, formulation are interchangeably used unless specified differently. Hence the term tablet, capsule, drageé, pellet, suppository, formulation pertains especially to all the components contained in one respective dosage unit or galenic unit or piece, which are comprised in a composition of the present invention.

The ingredients comprised in a composition of the present invention are now described with their contemplated function. Moreover the quantitative amount and the quality required is described, in particular if deemed important.

### VITAMIN C

Vitamin C is a well known water-soluble antioxidant. Humans depend on external sources of vitamin C to meet their vitamin C requirements. Vitamin C in the form of ascorbate is found in the aqueous humor of human eyes. Low plasma levels of Vitamin C might be associated with an increased risk of developing AMD (age related macular degeneration).
Vitamin C typically protects the retina against the side effects of light.
The EU recommended daily allowance (RDA) for vitamin C in the form of ascorbic acid is 60 mg.
The subject composition provides a daily dose of preferably 60 mg of vitamin C or ascorbic acid. As used herein, vitamin C is equal to ascorbic acid and vice versa.

Calcium ascorbate is the preferred source of vitamin C in a composition, e.g. capsule, although other sources such as for example free ascorbic acid or sodium ascorbate could alternatively be used.
In a preferred aspect a pharmaceutical composition of the present invention contains 91.5 mg calcium ascorbate, which amount includes 25% overages for degradation.
On a total daily dosage vitamin C is typically present in an amount of from 30 - 90 mg, more preferably from 35 - 85 mg, also preferably from 40 - 80 mg, and even more preferably from 55 - 70 mg.

### VITAMIN E

Vitamin E is also a well-known lipid soluble antioxidant and may prevent oxidation of polyunsaturated fatty acids, e.g. in cell membranes by scavenging free radicals. Vitamin E can work synergistically with vitamin C in protecting vital cell function from normal oxidants. The DHA (docohexanoic acid) supplementation of the present invention requires supplementation in Vitamin E that plays a protective role on the membranous lipids. The EU recommended daily allowance (RDA) for vitamin E is at present set to 10 mg. However recent studies suggest that higher amounts might be rather beneficial, and hence it is contemplated that this recommendation is subject to changes.
Vitamin E is readily oxidized thereby significantly reducing its activity during periods of storage prior to ingestion. Once ingested, vitamin E is stored within the body and can contribute to the total body pool of vitamin E for up to one year.
Preferably the subject composition provides approximately 20 mg of vitamin E per composition, e.g. per capsule.

As used herein, d,l-alpha tocopheryl acetate stands for vitamin E and is the preferred source of vitamin E in the subject composition, e.g. capsule, although other sources of vitamin E, such as for example d,l-alpha tocopheryl acetate and/or d-alpha-tocopheryl acid succinate, may be used in the alternative.
1.0 mg of vitamin E is equal to 1 IU of d,l-alpha tocopheryl acetate. Alternatively, 1 mg d-alpha tocopheryl acetate corresponds to 1.5 IU d,l-alpha tocopheryl acetate.
In a preferred aspect a pharmaceutical composition of the present invention contains approximately 31 mg d,l-alpha tocopheryl acetate, which amount includes 5% overages for degradation.
On a total daily dosage vitamin E is typically present in an amount of from 5 - 35 mg, especially from 10 - 30 mg, and in particular from 15 - 25 mg.

### ZINC

Zinc is important in maintaining the health of an eye's retina and is an essential part of more than 100 enzymes involved in digestion, metabolism, reproduction and wound healing. The RDA for zinc is approximately 15 mg.
Zinc plays a role as a cofactor for enzymes that directly participates in oxidant defense.
Zinc concentrations in the retina and choroid are among the highest in the body.

Studies have shown that Zinc slows visual field loss in AMD patients.
Preferably, the subject composition provides approximately 15 mg zinc per composition, e.g. per capsule.
Zinc is preferred in the form of zinc oxide in subject tablets due to the fact zinc oxide provides the most concentrated form for elemental zinc and is well tolerated in the digestive system. However, other forms of zinc such as for example zinc gluconate, zinc citrate, zinc acetate, zinc chloride, zinc lactate, or zinc sulfate may alternatively be used or be used in combination with zinc oxide in the subject composition. As used herein, zinc refers to a zinc salt, and more preferably to zinc oxide, zinc chloride or zinc gluconate, most preferably to zinc gluconate.
In a preferred aspect a pharmaceutical composition of the present invention contains 12.4 mg zinc oxide.
For zinc, typically no overages are required, since zinc is not subject to degradation.
On a total daily dosage zinc is typically present in an amount of from 3 - 20 mg, more preferably from 6 - 17 mg, and in particular from 8 - 13 mg.

### COPPER

Copper, like zinc, is another important cofactor for metalloenzymes, and is a second necessary cofactor for superoxide bismuthase. The total recommended daily dosage of copper is typically approximately 0.9 mg copper per day.
Copper in the form of cupric oxide is preferred in the present compositions, although other forms of copper such as for example copper carbonate or copper gluconate may alternatively be used or used in combination with cupric oxide in the subject composition.
In a preferred aspect a composition of the present invention contains 0.45 mg cupric carbonate, which corresponds to approximately 0.25 mg labeled copper.
For copper, typically no overages are required, since copper is not subject to degradation.

In another preferred aspect of this invention, a composition of the present invention contains no copper. While applicant wishes not to bound by any theory it is believed that the above described function of copper might be effected by other ingredients., hence copper is absent in said other preferred aspect.

### LUTEIN

Lutein, is a carotenoid. Lutein is also an antioxidant found in the retina of healthy eyes. Lutein and zeaxanthine are xanthophylls, belonging to the group of carotenoids.
Lutein and zeaxanthine are pigments found in retina; mostly in the macula area , where they play a filter role from blue light and probably an anti oxidant role. These pigments are not synthesized in vivo , thus an external (food) supplementation is required for the macular pigment composition.
Lutein typically contains naturally also zeaxanthin.
Epidemiologic studies suggests that lutein consumption might be inversely related to eye diseases such as AMD. Studies in human show that lutein supplementation results in increased macular pigment.
It appears from our studies that the supplementation of 10 mg / day decreases the AMD incidence. Therefore, the subject composition preferably provides preferably 10 mg of pure lutein per composition, e.g. per capsule.
Lutein in the form of pure Flora Glo (supplier for example Roche) might be used. This source provides crystalline lutein and zeaxanthin, from marigold oleoresins extracted from marigold flowers (tagetes erecta). Lutein, zeaxanthin and other carotenoïds represent, according to their label, 80 % of the weight of the raw material called "FloraGLO crystalline Lutein". This is taken into account when provided in a composition of the present invention.

In a preferred aspect a pharmaceutical composition of the present invention contains 55 mg lutein (20% in safflower oil), which amount includes 10% overages for degradation.

Also preferably, an addressed composition contains approximately 1 - 20 mg, more preferably 3 - 17 mg, and even more preferably 7 - 14 mg lutein per day.

### ZEAXANTHIN

Zeaxanthin, like lutein is a carotenoid. Zeaxanthin is also an antioxidant found in the retina of healthy eyes. Preferably a total daily dosage may range from approximately 100 to 2000 microgram (0.1 - 2 mg) depending upon whether zeaxanthin is used to supplement or substitute and/or lutein.
In a preferred aspect a pharmaceutical composition of the present invention contains 800 microgram zeaxanthin as part of the lutein supplementation.

### OMEGA-3-FATTY ACIDS

Fatty acids from the omega 3 group are mainly eicosapentaenoic acid (EPA) and docosahexaenoic acid (DHA). These fatty acids modify for example the composition with respect to the membranous permeability. They may also modify the distribution of membranous proteinic receptor.
Thus the protective role of DHA towards AMD may emerge from different mechanisms such as improving the rhodopsine (retinal pigment) regeneration at the pair pigmentary epithelium - photoreceptor level and/or may play a role in the setting up of a lipid background that would improve the rhodopsine activity.

Preferred examples of omega-3-fatty acids are docohexaenoic acid (DHA) and eicosapentaenoic acid (EPA).

In a preferred aspect the daily dosage of omega-3 fatty acid is approximately 160 mg/day. The supplementation in both EPA /DHA might be beneficial , because the retina contains DHA, and EPA is described as the physiologic precursor of DHA.
The preferred source for omega-3 fatty acid is fish oil. The source of the fish oil may have a signficant influence on the content of DHA and EPA.
Preferably the subject composition provides approximately 160 mg of an omega-3-fatty acid per tablet, more preferably 240 mg of fish oil, having a content of about 70% DHA and variable amounts of EPA. The ratio of DHA / EPA varies to a great extent depending on the source of the fish oil.
Preferably the subject composition provides approximately 100 - 300 mg, more preferably 150 - 250 mg omega-3 fatty acid per day.
In a preferred aspect a pharmaceutical composition of the present invention contains 160 mg omega-3-fatty acid (240 mg fish oil enriched.

### Advantages of the present invention:

Free radicals initiate local molecular instability leading to cellular damage. Formation of free radicals is caused by metabolism, sunlight (blue-light spectrum), other free radicals, lack of antioxidants, and other factors. This process may lead to deteriorating vision due to age-related macular degeneration (AMD) and/or diabetic retinopathy (DR).
Antioxidant supplementation with certain nutrients may counteract the chain reaction of free radical damage by neutralizing the electron imbalance. Replenishing the antioxidant potential of the retina may effectively decrease oxidant stress and slow or decrease retinal deterioration.
There is a strong scientific evidence that the ingredients of the present invention provide help to patients with AMD, i.e. reduced risk of developing advanced AMD and reduced risk of vision loss.
In addition, the formulation of the present invention may not only treat but also prevent retinal degeneration.
Another advantage of this invention is the use of a combination of omega 3 fatty acids with specific, more stable vitamin molecules and also specific, more stable zinc and copper containing molecules.
Specifically, antioxidant vitamins (Vit. C and E) and minerals (Zn and/or Cu) may block the damage of the eye by free radicals. Additionally Lutein and/or Zeaxanthin are believed to rebuild the ocular pigment density of the macula, thereby providing protection against radiation damage of the retina. Omega 3 fatty acids containing high amounts of docosahexaeonic acid (DHA) and eicosapentaeonic acid (EPA) are contemplated of triggering / maintaining the levels of DHA in the photoreceptor cells protecting the ability of the eye to translate light impulses into nervous inputs for the brain (retina's ability to process images). They might also protect the photoreceptor cells from cell death. EPA is contemplated of inhibiting COX1 and COX2 activity which may control / reduce inflammatory events in the eye.

The compositions of the present invention typically exhibit highly improved stability, which improved stability ensures an better treatment procedure since stable formulations provide reproducible treatments.
This above stability is improved by the following measures:
The compounds containing metal ions are typically selected such that they are not water soluble, since water soluble metal compounds are typically responsible for the decomposition of the other active ingredients, especially the vitamins.
In case of the vitamins also the more stable compounds, i.e. alpha-tocopheryl acetate instead of alpha-tocopherol and calcium asorbate instead of ascorbic acid are used for the preparation of the compositions. This unique combination produced a synergistic stability improvement of the compositions in accordance of the present invention.

Manufacturing / Preparation
The compositions, capsules, formulations or tablets of the present invention are prepared in a manner known *per se*, for example by means of conventional mixing, granulating, coating, dissolving or lyophilizing processes.

The ingredients of the compositions may be sterilized, e.g. batch-wise, ingredients-wise as deemed appropriate and/or may comprise excipients, for example stabilizers, coloring agents, firming agents, wetting agents and/or emulsifiers, solubilizers, salts for regulating osmotic pressure and/or buffers and are prepared in a manner known *per se*, for example by means of conventional dissolving and lyophilizing processes. The said solutions or suspensions may comprise viscosity-increasing agents, typically sodium carboxymethylcellulose, carboxymethylcellulose, dextran, polyvinylpyrrolidone, or gelatins, or also solubilizers, for example Tween 80 [polyoxyethylene(20)sorbitan mono-oleate; trademark of ICI Americas, Inc, USA].

Suitable carriers are especially fillers, such as sugars, for example lactose, saccharose, mannitol or sorbitol, cellulose preparations, and/or calcium phosphates, for example tricalcium phosphate or calcium hydrogen phosphate, and also binders, such as starches, for example corn, wheat, rice or potato starch, methylcellulose, hydroxypropyl methylcellulose, sodium carboxymethylcellulose, and/or polyvinylpyrrolidone, and/or, if desired, disintegrators, such as the above-mentioned starches, also carboxymethyl starch, cross-linked polyvinylpyrrolidone, alginic acid or a salt thereof, such as sodium alginate. Additional excipients are especially flow conditioners and lubricants, for example silicic acid, talc, stearic acid or salts thereof, such as magnesium or calcium stearate, and/or polyethylene glycol, or derivatives thereof.

Compositions for oral administration also include hard or soft capsules consisting of gelatin, sealed capsules consisting of gelatin and a plasticizer, such as glycerol or sorbitol. The hard capsules may contain the active ingredient in the form of granules, for example in admixture with fillers, such as corn starch, binders, and/or glidants, such as talc or magnesium stearate, and optionally stabilizers. In soft capsules, the active ingredient is preferably dissolved or suspended in suitable liquid excipients, such as fatty oils, paraffin oil or liquid polyethylene glycols or fatty acid esters of ethylene or propylene glycol, to which stabilizers and detergents, for example of the polyoxyethylene sorbitan fatty acid ester type, may also be added.

## Claims

1. A composition comprising on a daily dosage basis:
approximately from 40 - 80 mg, preferably 40 - 60 mg of vitamin C;
approximately from 15 - 25 mg, preferably 15 - 20 mg of vitamin E
approximately from 7 - 14 mg , preferably 7 - 11 mg of lutein;
approximately of 8 - 15 mg, preferably 8 - 13 mg of zinc;
approximately 120 - 200 mg omega 3 fatty acid, and
optionally approximately 0.1 - 0.3 mg, preferably 0.25 mg of copper.

2. A composition of claim 1, wherein said copper is present.

3. A composition of claim 1, wherein said copper is absent.

4. Composition of claim 1, comprising zeaxanthin, preferably in an amount of approximately 100 to 2000 microgram (0.1 -2 mg), more preferably in an amount of approximately 100 to 1000 microgram (0.1 -1 mg)

5. Composition of claim 1, wherein said omega 3 fatty acid comprises docosahexaeonic acid (DHA) and eicosapentaenoic acid (EPA).

6. Composition of claim 1, wherein said omega 3 fatty acid stems from fish oil with a content of 70% by weight of DHA.

7. Composition of claim 1 wherein said vitamin C comprises or substantially consists of calcium ascorbate, said vitamin E comprises or substantially consists of d,l alpha tocopheryl acetate, said zinc comprises or substantially consists of zinc oxide, and said copper comprises or substantially consists of basic cupric carbonate.

8. A composition of claim 1, which is a tablet, capsule or pellet, and contains:
| | |
|---|---|
| 240 mg Fish oil with 70% DHA | label 160 mg Omega 3 fatty acids |
| (comprising 130 mg DHA and 11 mg EPA) | |
| 91.5mg Calcium ascorbate | label 60 mg Vitamin C |
| 31.343mg d,l Alpha-tocopheryl acetate | label 20 mg Vitamin E |
| 55mg Lutein 20% in safflower oil Zeaxanthine) | label 10 mg Lutein ( comprising 0.8 mg |
| 12.447mg Zinc oxide | label 10 mg Zinc |
| 0.45mg Cupric carbonate basic | label 0.25 mg Copper. |

9. Use of a composition in accordance to any of the preceding claims in the preparation of a medicament for the treatment and/or prevention of age related macular degeneration (AMD) and/or diabetic retinophathy (DR).

10. Use of claim 9, wherein said treatment and/or prevention is reducing the risk of developing advanced AMD and reducing the risk of vision loss

11. Method of treating and/or preventing AMD and/or DR in a subject being in need thereof, comprising administering an effective amount of a composition in accordance to any of the preceding claims.

12. Method of manufacturing a stable composition of claim 1, which method comprises admixing in a conventional manner vitamin C which comprises or substantially consists of calcium ascorbate, vitamin E which comprises or substantially consists of d,l alpha tocopheryl acetate, zinc which comprises or substantially consists of zinc oxide, optionally copper which, if present, comprises or substantially consists of basic cupric carbonate, and an omega 3 fatty acid which comprises or substantially consists of fish oil containing 70% of DHA.

13. Composition, method of manufacture, method of improving the stability, method of treatment or use substantially as hereinbefore defined and/or described.
